(19) Europäisches Patentamt
European Patent Office
Office européen des brevets

(11) **EP 1 968 708 B1**

(12) **EUROPEAN PATENT SPECIFICATION**

(45) Date of publication and mention of the grant of the patent:
**07.05.2014 Bulletin 2014/19**

(51) Int Cl.:
*A61Q 1/02* *(2006.01)*    *A61K 8/06* *(2006.01)*
*A61K 8/31* *(2006.01)*    *A61K 8/37* *(2006.01)*
*A61K 8/58* *(2006.01)*    *A61K 8/33* *(2006.01)*

(21) Application number: **06841582.7**

(22) Date of filing: **21.12.2006**

(86) International application number:
**PCT/EP2006/070127**

(87) International publication number:
**WO 2007/077156 (12.07.2007 Gazette 2007/28)**

(54) **COSMETIC COMPOSITION COMPRISING AN OIL**

ÖLHALTIGE KOSMETISCHE ZUSAMMENSETZUNG

COMPOSITION COSMETIQUE COMPRENANT UNE HUILE

(84) Designated Contracting States:
**AT BE BG CH CY CZ DE DK EE ES FI FR GB GR HU IE IS IT LI LT LU LV MC NL PL PT RO SE SI SK TR**

(30) Priority: **03.01.2006 FR 0650010**
**25.01.2006 US 761759 P**

(43) Date of publication of application:
**17.09.2008 Bulletin 2008/38**

(73) Proprietor: **L'OREAL**
**92665 Asnieres (FR)**

(72) Inventor: **AUGUSTE, Frédéric**
**F-94550 Chevilly-Larue (FR)**

(74) Representative: **Duvert, Sandra**
**L'Oréal**
**D.I.P.I.**
**River PLaza**
**25-29 Quai Aulagnier**
**92600 Asnieres-sur-Seine (FR)**

(56) References cited:
**EP-A- 1 502 574    EP-A2- 1 210 929**
**FR-A- 2 854 063    FR-A1- 2 841 464**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention relates to a cosmetic composition for making up or caring for the skin, in the form of an emulsion comprising at least one specific oil, and also to a process for making up or caring for the skin.

The makeup composition may in particular be a foundation, an eyeshadow, a makeup rouge, a concealer product or a body makeup product.

The care composition may in particular be a care product for bodily and facial skin (a day cream), especially an antisun product or a skin colouring product (such as a self-tanning product).

Foundations may be in the form of anhydrous compositions or emulsions, and may have textures varying from fluid to solid.

Emulsions with an oily continuous phase, or water-in-oil emulsions, are often preferred on account of their good adhesion to the epidermis; their ease of application (play time) and their ability to form a uniform deposit on the skin. The makeup obtained is comfortable and does not dry out the skin.

However, these emulsions have a greasy feel and have the drawback of transferring: the makeup applied to the skin is liable to come off, at least partly, onto certain supports with which it is placed in contact, for instance an item of clothing or the skin.

To avoid this transfer phenomenon, it is known practice to introduce volatile solvents into these compositions in order for the oily phase to evaporate quickly. However, during the application of the composition to the skin, excessively rapid evaporation of the continuous phase of the emulsion results in an increase in the viscosity of the makeup, which then becomes difficult to spread on the skin. Poor spreading of the composition on the skin results in a sensation of discomfort for the user and the makeup obtained is not uniform.

There is thus a need for skin makeup or care compositions in water-in-oil emulsion form that spread easily without having a greasy feel and which form a uniform makeup deposit, that does not transfer.

The inventors have demonstrated that it is possible to obtain such compositions by introducing a specific oil into the liquid fatty phase of a water-in-oil emulsion.

One subject of the present invention is thus a Cosmetic composition for making up or caring for the skin, in the form of a water-in-oil emulsion comprising an aqueous phase, at least 5% by weight, relative to the total weight of the composition, of a dispersed solid phase and a liquid fatty phase having at least one non-volatile fraction, the liquid fatty phase comprising:

- at least two oils chosen from slow volatile oils with an evaporation rate of between 0.002 and 0.05 mg/cm$^2$/minute according to the protocol defined in the description and dry oils chosen from oils with a viscosity of less than or equal to 10 Cps, a surface tension of between 21 and 31 mN/m and an evaporation rate of less than 0.002 mg/cm$^2$/minute according to the protocol defined in the description,

the liquid fatty phase having an evaporation rate such that the weight ratio of the total mass of the liquid fatty phase after 10 minutes of drying according to the protocol defined in the description, ie $M = \sum_i m_i(t)$ at t=10

minutes, to the initial liquid fatty phase ie $M = \sum_i m_i(t)$ at t= 0 minutes, ranges from 0.75 to 1,

wherein
i represents each solvent
$m_1(t)$ represents the remaining mass of each solvent i per unit area at time t according to the following equations

$$m_i(t) = m_i(0) - v_i . t . \quad if \quad t < \frac{m_i(0)}{v_i}$$

$$m_i(t) = 0 \qquad if \qquad t \geq \frac{m_i(0)}{v_1}$$

wherein $v_1$ represents the evaporation rate measured according to the protocol defined in the description and expressed in mg of oil evaporated per unit area (cm$^2$) and per unit time (minutes).
and
the aqueous phase and the liquid fatty phase being such that the weight ratio R of:

1) the ratio of the mass, at a time t = 10 minutes, of the liquid fraction of the dispersed aqueous phase <u>to</u> the mass, at a time t = 10 minutes, of the liquid fraction of the continuous fatty phase measured according to the protocol defined in the description <u>divided by</u>

2) the ratio of the initial mass of the liquid fraction of the dispersed aqueous phase to the initial mass of the liquid fraction of the continuous fatty phase measured according to the protocol defined in the description, is less than or equal to 1,

characterized in that the dry oil is present in a content ranging from 50% to 100% by weight, preferably ranging from 70% to 99% by weight, more preferably ranging from 80% to 98% by weight and more preferentially ranging from 90% to 97% by weight relative to the total weight of the non-volatile fraction of the liquid fatty phase, and is chosen from:

- isopropyl myristate, isopropyl palmitate, 2-ethylhexyl benzoate, isodecyl neopentanoate, 2-ethylhexyl 2-ethylhexanoate, isononyl isononanoate,
- dicaprylyl ether, dicaprylyl carbonate,
- 2-diethylhexyl carbonate,

and mixtures thereof.

A subject of the invention is also a process for making up or caring for the skin, comprising the application to the skin of a composition as described above.

## Liquid fatty phase

The composition according to the invention comprises a liquid fatty phase consisting of a volatile fraction and a non-volatile fraction.

The term "non-volatile fraction of the liquid fatty phase" means all the non-volatile oils of the liquid fatty phase.

The term "volatile fraction of the liquid fatty phase" means all the volatile oils of the liquid fatty phase.

### Dry oils

The composition according to the invention may comprise at least one dry oil.

For the purposes of the present patent application, the term "dry oil" means an oil chosen from oils with a viscosity of less than or equal to 0.01 Pa.s (10 Cps) and especially ranging from 0.003 to 0.01 Pa.s, a surface tension of between 21 and 31 mN/m and an evaporation rate of less than 0.002 mg/cm$^2$/minute, and the dry oils are chosen from isopropyl myristate, isopropyl palmitate, 2-ethylhexyl benzoate, isodecyl neopentanoate, 2-ethylhexyl 2-ethylhexanoate, isononyl isononanoate,

- dicaprylyl ether, dicaprylyl carbonate (Cetiol CC),
- 2-diethylhexyl carbonate,

and mixtures thereof.

According to one preferred embodiment, the dry oil is isononyl isononanoate.

The dry oil(s) is present in the composition according to the invention in a content ranging from 50% to 100% by weight, preferably from 70% to 99% by weight, more preferentially from 80% to 98% by weight and more preferentially from 90% to 97% by weight relative to the total weight of the non-volatile fraction of the liquid fatty phase.

According to one particular embodiment, the dry oils may represent 100% of the non-volatile fraction of the liquid fatty phase.

The dry oil(s) may be present in the composition according to the invention in a content ranging from 1% to 95% by weight, preferably from 2% to 50% and more preferentially from 3% to 20% by weight relative to the total weight of the composition.

## Additional non-volatile oil

Besides the dry oil(s) defined above, the non-volatile fraction of the liquid fatty phase may comprise at least one additional non-volatile oil.

The term "non-volatile oil" means an oil remaining on the skin at room temperature and atmospheric pressure for at least several hours and especially having a vapour pressure of less than 0.13 Pa (0.01 mmHg).

According to one particular embodiment, the non-volatile oil is chosen from oils with an evaporation rate of less than 0.002 mg/cm$^2$/minute.

The term "additional non-volatile oil" means non-volatile oils other than the dry oils described above.

The additional non-volatile oils may be hydrocarbon-based oils especially of animal or plant origin, silicone oils, or mixtures thereof. The term "hydrocarbon-based oil" means an oil mainly containing hydrogen and carbon atoms and possibly oxygen, nitrogen, sulfur and/or phosphorus atoms.

The additional non-volatile oils may be chosen especially from non-volatile hydrocarbon-based oils, which may be fluorinated, and/or non-volatile silicone oils.

Non-volatile hydrocarbon-based oils that may especially be mentioned include:

- hydrocarbon-based oils of animal origin,

- hydrocarbon-based oils of plant origin, such as triglycerides consisting of fatty acid esters of glycerol, the fatty acids of which may have chain lengths ranging from $C_4$ to $C_{24}$, these chains possibly being linear or branched, and saturated or unsaturated; these oils are especially heptanoic or octanoic acid triglycerides, or alternatively wheat germ oil, sunflower oil, grape seed oil, sesame seed oil, corn oil, apricot oil, castor oil, shea oil, avocado oil, olive oil, soybean oil, sweet almond oil, palm oil, rapeseed oil, cottonseed oil, hazelnut oil, macadamia oil, jojoba oil, alfalfa oil, poppy oil, pumpkin oil, marrow oil, blackcurrant oil, evening primrose oil, millet oil, barley oil, quinoa oil, rye oil, safflower oil, candlenut oil, passionflower oil or musk rose oil; shea butter; or caprylic/capric acid triglycerides, for instance those sold by the company Stéarineries Dubois or those sold under the names Miglyol 810®, 812® and 818® by the company Dynamit Nobel,

- synthetic ethers containing from 10 to 40 carbon atoms, other than those corresponding to the definition of the dry oils, such as diisocetyl ether;

- linear or branched hydrocarbons of mineral or synthetic origin, such as petroleum jelly, polydecenes, hydrogenated polyisobutene such as Parleam®, squalane and liquid paraffins, and mixtures thereof,

- synthetic esters, other than those corresponding to the definition of the dry oils, for instance oils of formula $R_1COOR_2$ in which $R_1$ represents a linear or branched fatty acid residue containing from 1 to 40 carbon atoms and $R_2$ represents a hydrocarbon-based chain, which is especially branched, containing from 1 , to 40 carbon atoms, on condition that $R_1 + R_2 \geq 10$, for

instance Purcellin oil (cetostearyl octanoate), $C_{12}$ to $C_{15}$ alkyl benzoates, hexyl laurate, diisopropyl adipate, 2-ethylhexyl palmitate, isostearyl isostearate, 2-hexyldecyl laurate, 2-octyldecyl palmitate, 2-octyldodecyl myristate, and heptanoates, ocatanoates, decanoates or ricinoleates of alcohols or of polyalcohols, for instance propyl.ene glycol dioctanoate; hydroxylated esters, for instance isostearyl lactate, diisostearyl malate or 2-octyldodecyl lactate; polyol esters and pentaerythritol esters,

- fatty alcohols that are liquid at room temperature, with a branched and/or unsaturated carbon-based chain containing from 12 to 26 carbon atoms, for instance octyldodecanol, isostearyl alcohol, oleyl alcohol, 2-hexyldecanol, 2-butyloctanol and 2-undecylpenta-decanol,

- higher fatty acids such as oleic acid, linoleic acid and linolenic acid, and mixtures thereof.

The non-volatile silicone oils that may be used in the composition according to the invention may be non-volatile polydimethylsiloxanes (PDMS), polydimethylsiloxanes comprising alkyl or alkoxy groups that are pendent and/or at the end of a silicone chain, these groups each containing from 2 to 24 carbon atoms, phenyl silicones, for instance phenyl trimethicones, phenyl dimethicones, phenyltrimethylsiloxydiphenyl-siloxanes, diphenyl dimethicones and diphenylmethyldi-phenyltrisiloxanes, and mixtures thereof.

The additional non-volatile oils may be present in the composition in a content ranging from 0.5% to 50% by weight, preferably from 1% to 30% by weight and more preferentially from 2% to 20% by weight relative to the total weight of the non-volatile fraction of the liquid fatty phase.

The additional non-volatile oils may be present in the composition in a content of less than or equal to 7%, in particular ranging from 0.5% to 5% by weight, preferably from 1% to 5% by weight and more preferentially from 1% to 2% by weight relative to the total weight of the composition.

The liquid fatty phase of the composition according to the invention comprises a volatile fraction comprising volatile oils chosen from slow volatile oils and/or fast volatile oils.

For the purposes of the invention, the term "volatile oil" means any oil capable of evaporating on contact with the skin, at room temperature and atmospheric pressure. The volatile oils of the invention are volatile cosmetic oils, which are liquid at room temperature, with a non-zero vapour pressure, at room temperature and atmospheric pressure, in particular ranging from 0.13 Pa to 40 000 Pa (0.001 to 300 mmHg) and preferably ranging from 1.3 to 1300 Pa (0.01 to 10 mmHg).

In particular, the volatile oils are chosen from oils with an evaporation rate of greater than or equal to 0.002 mg/cm$^2$/minute.

The composition according to the invention may especially comprise a slow volatile oil.

For the purposes of the present invention, the term "slow volatile oil" means an oil chosen from volatile oils as defined above, with an evaporation rate of between 0.002 and 0.05 mg/cm$^2$/minute.

In particular, the slow volatile oil(s) may be chosen from isohexadecane, cyclohexasiloxane, diethyldodecane (for example Cetiol DD from the company Cognis) and hexyl trimethicone, and mixtures thereof.

The slow volatile oil(s) may be present in the composition according to the present invention in a content ranging from 1% to 95% by weight, preferably ranging from 2% to 50% by weight and more preferentially from 3% to 20% by weight relative to the total weight of the composition.

The composition according to the invention may comprise a fast volatile oil.

For the purposes of the present invention, the term "fast volatile oil" means an oil chosen from volatile oils as defined above, with an evaporation rate of greater than or equal to 0.05 mg/cm$^2$/minute, in particular ranging from 0.05 to 200 mg/cm$^2$/minute, especially greater than or equal to 0.054 mg/cm$^2$/minute, in particular ranging from 0.054 to 100 mg/cm$^2$/minute and preferentially ranging from 0.054 to 30 mg/cm$^2$/minute.

The fast volatile oil may be chosen from hydrocarbon-based volatile oils, silicone volatile oils and fluoro volatile oils, and mixtures thereof.

The term "hydrocarbon-based oil" means an oil mainly containing hydrogen and carbon atoms, and possibly oxygen, nitrogen, sulfur and/or phosphorus atoms.

The volatile hydrocarbon-based oils may be chosen from hydrocarbon-based oils containing from 8 to 16 carbon atoms, and especially branched $C_8$-$C_{16}$ alkanes, for instance $C_8$-$C_{16}$ isoalkanes of petroleum origin (also known as isoparaffins), for instance isododecane (also known as 2,2,4,4,6-pentamethylheptane), isodecane, and, for example, the oils sold under the trade names Isopar$^®$ or Permethyl$^®$.

Volatile oils that may also be used include volatile silicones, for instance volatile linear or cyclic silicone oils, especially those with a viscosity $\leq$ 5 centistokes ($5 \times 10^{-6}$ m$^2$/s) and especially containing from 2 to 10 silicon atoms and preferably from 2 to 7 silicon atoms, these silicones optionally comprising alkyl or alkoxy groups containing from 1 to 10 carbon atoms. As volatile silicone oils that may be used in the invention, mention may be made especially of octamethylcyclotetrasiloxane, decamethylcyclopentasiloxane, heptamethylhexyltrisiloxane, heptamethyl-octyltrisiloxane, hexamethyldisiloxane, octamethyltrisiloxane, decamethyltetrasiloxane and dodecamethyl-pentasiloxane, and mixtures thereof.

The volatile fluoro oil generally does not have a flash point.

Volatile fluoro oils that may be mentioned include nonafluoroethoxybutane, nonafluoromethoxybutane, decafluoropentane, tetradecafluorohexane and dodecafluoropentane, and mixtures thereof.

According to one particular embodiment, the content of fast volatile solvents (fast volatile oil and fast volatile solvents present in the aqueous phase) may be less than 64% by weight, preferably less than 60% by weight and more preferentially less than 50% by weight relative to the total weight of the composition.

In particular, the content of fast volatile solvents in the composition according to the invention may range from 1% to 64% by weight, preferably from 2% to 50% by weight, more preferentially from 3% to 30% by weight and more preferably from 4% to 20% by weight relative to the total weight of the composition.

The volatile oils (slow and fast volatile oils) may be present in the composition in a content ranging from 1% to 50% by weight relative to the total weight of the composition, preferably ranging from 3% to 30% by weight and more preferentially ranging from 5% to 20% by weight relative to the total weight of the composition.

The composition according to the invention comprises at least 15% by weight of fatty phase (liquid and solid), relative to the total weight of the composition.

In particular, the total fatty phase (liquid and solid) is present in the compositions according to the invention in a content ranging from 15% to 95% by weight, preferably 20% to 90% by weight and more preferentially 25% to 85% by weight relative to the total weight of the composition.

## Evaporation profile

The nature and content of the various oils defined above may be chosen by a person skilled in the art such that the composition according to the invention has a particular evaporation profile.

The composition according to the invention comprises (may comprise) a liquid fatty phase such that the calculated weight ratio of the liquid fatty phase after 10 minutes of drying, to the initial liquid fatty phase, ranges from 0.75 to 1, preferably from 0.75 to 0.9 and more preferentially from 0.8 to 0.9.

**Calculation of the evaporation rate**

Measurement of the evaporation rate:

The calculations of the mass of liquid fatty phase after 10 minutes of drying may be made from the specific evaporation rates of the oils, measured at 20°C according to the following protocol: 15 g of oil or of the mixture of oils to be tested are introduced into a crystallizing basin (diameter: 7 cm) placed on a balance which is in a chamber of about 0.3 $m^3$ of controlled temperature (25°C) and hygrometry (50% relative humidity).

The liquid is left to evaporate freely, without shaking it, ventilation being provided by means of a fan (spin speed 2700 rpm and dimensions 80 × 80 × 42 mm, for example the reference 8550 N from Papst-Motoren, the output corresponds to about 50 $m^3$/hour) placed vertically above the crystallizing basin containing the solvent, the vanes being directed towards the crystallizing basin and 20 cm from the bottom of the crystallizing basin.

The mass of oil remaining in the crystallizing basin is measured at regular intervals. The evaporation rates are expressed in mg of oil evaporated per unit area ($cm^2$) and per unit time (minutes).

In this approach, the "dry" oils and the non-volatile oils are considered as having zero evaporation rates.

Equations used:

For 100 mg of a composition comprising i solvents each having an evaporation rate $v_i$ (measured according to the protocol described above, expressed in mg of oil evaporated per unit area ($cm^2$) and per unit time (minutes)).

The solvents are introduced into the composition in an initial amount per unit area equal to $m_i(0)$ (expressed in mg per $cm^2$).

For each solvent, the remaining mass per unit area at a time t [$m_i(t)$] may be given by the following equations:

$$m_i(t) = m_i(0) - v_i.t. \quad if \quad t < \frac{m_i(0)}{v_i}$$

$$m_i(t) = 0 \quad if \quad t \geq \frac{m_i(0)}{v_i}$$

The total mass of *liquid* fatty phase is then given by the sum of all the individual masses $m_i(t)$ at each of the times:

$$M = \sum_i m_i(t)$$

The calculation is thus made for a time t = 10 minutes.

**Aqueous phase**

The composition according to the invention comprises an aqueous phase.

The composition according to the invention comprises water. The water may be a floral water such as cornflower water and/or a mineral water such as eau de Vittel, eau de Lucas or eau de La Roche Posay and/or a spring water.

The composition according to the invention, and especially the aqueous phase, may also comprise organic solvents that are water-miscible (at room temperature-25°C), for instance monoalcohols containing from 2 to 6 carbon atoms such as ethanol or isopropanol; polyols especially containing from 2 to 20 carbon atoms, preferably containing from 2 to 10 carbon atoms and preferentially containing from 2 to 6 carbon atoms, such as glycerol, propylene glycol, butylene glycol, pentylene glycol, hexylene glycol, dipropylene glycol or diethylene glycol; glycol ethers (especially containing from 3 to 16 carbon atoms) such as mono-, di- or tripropylene glycol ($C_1$-$C_4$)alkyl ethers or mono-, di- or triethylene glycol ($C_1$-$C_4$)alkyl ethers, and mixtures thereof.

The liquid fraction of the aqueous phase especially comprises water and water-miscible organic solvents.

The aqueous phase may also comprise stabilizers, for instance sodium chloride, magnesium dichloride and magnesium sulfate.

The aqueous phase may also comprise any water-soluble or water-dispersible compound that is compatible with an aqueous phase, such as gelling agents, film-forming polymers, thickeners and surfactants, and mixtures thereof.

Water, and water-miscible solvents with an evaporation rate of greater than 0.05 mg/cm$^2$/minute, are considered as fast volatile components of the aqueous phase within the meaning of the present patent application.

Preferably, the aqueous phase may be present in the composition according to the invention in a content ranging from 0.5% to 50% by weight, preferably ranging from 1% to 40% by weight and preferentially ranging from 5% to 30% by weight relative to the total weight of the composition.

Preferably, the water may be present in the composition according to the invention in a content ranging from 0.5% to 50% by weight, preferably ranging from 1% to 40% by weight and preferentially ranging from 5% to 30% by weight relative to the total weight of the composition.

**Dispersed solid phase**

The composition according to the invention may comprise a dispersed solid phase.

For the purposes of the present patent application, the term "dispersed solid phase" means any compound which, after evaporation of all the volatile components (essentially the oils, the water and the water-miscible organic solvents), is in the form of solid particles.

The term "solid particles"' means particles which are in solid form at 25°C and at atmospheric pressure.

According to a first aspect, these solid particles may comprise (in particular are formed from) a crystalline or semi-crystalline material that is solid at room temperature (25°C) with a first-order phase transition, of melting (changing from the solid state to the liquid state) or of evaporation (changing from the solid state to the gaseous state) of greater than 100°C, preferably greater than 120°C and better still greater than 150°C.

The melting or evaporation temperature of the first material may be measured according to ASTM standard E794-98.

For the purposes of the invention, the term "semi-crystalline material" means a material, especially a polymer, comprising a crystallizable part and an amorphous part with a first-order reversible phase-change temperature, in particular of melting (solid-liquid transition).

The material of the said solid particles may be a mineral material that may be chosen from silica, glass, diamond, copper, boron nitride, ceramics, metal oxides, especially iron oxides, for instance black iron oxide, red iron oxide or yellow iron oxide, titanium oxides, micas, alumina, or certain semi-crystalline polymers, and mixtures thereof.

According to a second aspect, these solid particles may comprise (in particular may be formed from) an amorphous material with a glass transition temperature of greater than or equal to 60°C (especially ranging from 60°C to 800°C), advantageously greater than or equal to 80°C (especially ranging from 80°C to 700°C) and preferably greater than or equal to 100°C (especially ranging from 100°C to 500°C). The glass transition temperature may be measured by DSC (differential scanning calorimetry) according to ASTM standard D3418-97.

An amorphous material that may be used is a polymer that is not film-forming at a temperature of less than or equal to 40°C, and which has a glass transition temperature as described above.

The term "polymer that is not film-forming at a temperature of less than 40°C" means a polymer that is not capable of forming, by itself or in the presence of an auxiliary film-forming agent, a continuous film that adheres to a support, especially to keratin materials, at a temperature of less than or equal to 40°C.

The term "auxiliary film-forming agent" means the plasticizers and coalescers known to those skilled in the art for promoting the film formation of polymers.

Amorphous polymers with a glass transition temperature of greater than or equal to 60°C that may be used include free-radical polymers or polycondensates having this defined glass transition temperature.

Free-radical polymers that may be mentioned include:

- ethylene polymers, especially cycloethylene or naphthylethylene polymers; .
- propylene polymers, especially hexafluoropropylene polymers;
- acrylic polymers, especially acrylic acid, dimethyladamantyl acrylate or chloroacrylate polymers;
- acrylamide polymers;
- (meth)acrylonitrile polymers;
- styrene, acetylstyrene, carboxystyrene or chloro-methylstyrene polymers.

Polycondensates that may be mentioned include polycarbonates, polyurethanes, polyesters, polyamides, polysulfones, polysulfonamides and carbohydrates, for instance amylose triacetate.

Solid particles that may be used include the aqueous dispersions of non-film-forming polymer sold under the names Joncryl® SCX 8082, Joncryl® 90 by the company Johnson Polymer, Neocryl® XK 52 by the company Avecia Resins, and Rhodopas® 5051 by the company Rhodia Chimie.

According to one particular embodiment, the solid particles are essentially formed from the said amorphous material described above.

The solid particles according to the present invention may be non-hollow solid particles, hollow particles or porous particles.

Without wishing to be limited by a list, the dispersed solid phase of the composition according to the invention may comprise pigments, nacres and/or fillers.

Pigments and nacres

The term "pigments" should be understood as meaning white or coloured, mineral or organic particles, which are insoluble in the liquid organic phase, and which are intended to colour and/or opacify the composition.

The term "nacres" should be understood as meaning iridescent particles, especially produced by certain molluscs in their shell, or else synthesized, which are insoluble in the medium of the composition.

The pigments may be chosen from mineral pigments, organic pigments and composite pigments (i.e. pigments based on mineral and/or organic materials).

The term "pigments" should be understood as meaning mineral or synthetic particles of any shape, having an optical effect, which are insoluble in the medium of the composition irrespective of the temperature at which the composition is manufactured.

The pigments may be chosen from monochromatic pigments, lakes, nacres and optical-effect pigments, for instance reflective pigments and goniochromatic pigments.

The mineral pigments may be chosen from metal oxide pigments, mica coated with titanium dioxide mica coated with bismuth oxychloride, titanium mica coated with iron oxide, titanium mica coated with ferric blue, titanium mica coated with chromium oxide, iron oxides, titanium dioxide, zinc oxides, cerium oxide, zirconium oxide or chromium oxide; manganese violet, Prussian blue, ultramarine blue, ferric blue, bismuth oxychloride, coloured nacreous pigments such as titanium mica with iron oxides, titanium mica especially with ferric blue or with chromium oxide, titanium mica with an organic pigment of the abovementioned type and also nacreous pigments based on bismuth oxychloride, and mixtures thereof.

The organic particles intended to be coated may be, for example:

- cochineal carmine,
- organic pigments of azo, anthraquinone, indigoid, xanthene, pyrene, quinoline, triphenylmethane or fluorane dyes;
- organic lakes or insoluble sodium, potassium, calcium, barium, aluminium, zirconium, strontium or titanium salts of acidic dyes such as azo, anthraquinone, indigoid, xanthene, pyrene, quinoline, triphenylmethane or fluorane dyes. These dyes generally comprise at least one carboxylic or sulfonic acid group;
- melanin pigments.

Among the organic pigments that may be mentioned are D&C Blue No. 4, D&C Brown No. 1, D&C Green No. 5, D&C Green No. 6, D&C Orange No. 4, D&C Orange No. 5, D&C Orange No. 10, D&C Orange No. 11, D&C Red No. 6, D&C Red No. 7, D&C Red No. 17, D&C Red No. 21, D&C Red No. 22, D&C Red No. 27, D&C Red No. 28, D&C Red No. 30, D&C Red No. 31, D&C Red No. 33, D&C Red No. 34, D&C Red No. 36, D&C Violet No. 2, D&C Yellow No. 7, D&C Yellow No. 8, D&C Yellow No. 10, D&C Yellow No. 11, FD&C Blue No. 1, FD&C Green No. 3, FD&C Red No. 40, FD&C Yellow No. 5, FD&C Yellow No. 6.

The organic lake may also be supported on any compatible support such as a mineral support, for instance alumina, clay, zirconia or metal oxide, especially zinc oxide or titanium oxide, talc, calcium carbonate or barium sulfate particles. Preferably, the mineral support is chosen from alumina, titanium oxide and barium sulfate.

The organic lake may also be supported on a support such as rosin or aluminium benzoate.

Among the organic lakes that may be mentioned in particular are those known under the following names: D & C Red Aluminium lake; D & C Blue Aluminium lake; D & C Green Aluminium lake; D & C Orange Aluminium lake; D & C Yellow Aluminium lake.

The chemical compounds corresponding to each of the organic pigments mentioned above are mentioned in the publication "International Cosmetic Ingredient Dictionary and Handbook", 1997 edition, pages 371 to 386 and 524 to 528, published by "The Cosmetic, Toiletry and Fragrance Association", the content of which is incorporated into the present patent application by reference.

The melanin pigments that may be used according to the invention are in particular:

- melanin pigments derived from natural or synthetic sources, and which may be obtained: (A) via oxidation of at least one indole or indoline compound, or (B) via oxidative or enzymatic polymerization of melanin precursors, or (C) via extraction of melanin from substances containing it, or (D) by culturing microorganisms. Such melanin pigments are especially described in documents EP-A-518 773, WO-A-93/13744 and WO-A-93/13745.

The pigments may be present in the composition according to the invention in a content ranging from 0.1% to 50% by weight, preferably ranging from 1% to 30% by weight and preferentially ranging from 5% to 20% by weight relative to the total weight of the composition.

The nacres may be chosen from white nacreous pigments such as mica coated with titanium or with bismuth oxychloride, coloured nacreous pigments such as titanium mica coated with iron oxides, titanium mica especially with ferric blue or with chromium oxide, titanium mica with an organic pigment of the abovementioned type and also nacreous pigments based on bismuth oxychloride.

Fillers

Besides the pigments and/or nacres, the dispersed solid phase of the composition according to the invention may comprise fillers.

The term "fillers" should be understood as meaning colourless or white, mineral or synthetic particles of any shape, which are insoluble in the medium of the composition- irrespective of the temperature at which the composition is manufactured.

The additional fillers may be mineral or organic and of any shape, platelet-shaped, spherical or oblong, irrespective of the crystallographic form (for example cubic, hexagonal, orthorhombic, etc.), solid, hollow or porous. Mention may be made of talc, mica, silica, kaolin, polyamide (Nylon®) powders, poly-β-alanine powders, polyethylene powders, polyurethane powders such as the powdered copolymer of hexamethylene diisocyanate and of trimethylol hexyl lactone sold under the name Plastic Powder D-400 by the company Toshiki, tetrafluoroethylene polymer (Teflon®) powders, lauroyllysine, starch, boron nitride, hollow polymer microspheres such as those of polyvinylidene chloride/ acrylonitrile, for instance Expancel® (Nobel Industrie) or of acrylic acid copolymers, silicone elastomer powders, silicone resin powders, in particular silsesquioxane powders (silicone resin powders described especially in patent EP 293 795; for example Tospearls® from Toshiba), polymethyl methacrylate particles, precipitated calcium carbonate, magnesium carbonate, magnesium hydrogen carbonate, hydroxyapatite, hollow silica microspheres, glass or ceramic microcapsules, metal soaps derived from organic carboxylic acids containing from 8 to 22 carbon atoms and preferably from 12 to 18 carbon atoms, for example zinc stearate, magnesium stearate, lithium stearate, zinc laurate or magnesium myristate; barium sulfate, and mixtures thereof.

The fillers may be present in the composition in a content ranging from 0.05% to 30% by weight, preferably ranging from 0.5% to 20% by weight and more preferentially ranging from 2% to 10% by weight relative to the total weight of the composition.

**Volume fraction of the dispersed solid phase**

According to one preferred embodiment, the dispersed solid phase may be present in the composition in a content such that, after evaporation of all the volatile components, the volume fraction of the dispersed solid phase is greater than or equal to 20% by volume and preferably ranges from 20% to 98% by volume relative to the total volume of the composition.

In particular, the.dispersed solid phase may be present in the composition in a content such that, after evaporation of all the volatile components, the volume fraction of the dispersed solid phase is greater than or equal to 55% by volume, preferably greater than or equal to 60% by volume and more preferentially greater than or equal to 65% by volume relative to the total volume of the non-volatile fraction of the composition.

Even more particularly, the volume fraction of the dispersed solid phase may range from 55% to 98% by volume, preferably from 60% to 90% by volume and more preferentially from 65% to 85% by volume relative to the total volume of the non-volatile fraction of the composition.

The dispersed solid phase is present in the compositions according to the invention in a content of at least 5% by weight relative to the total weight of the composition.

In particular, the dispersed solid phase is present in a content ranging from 5% to 50% by weight, better still from 10% to 45% by weight and even better still from 15% to 40% by weight relative to the total weight of the composition.

**Calculation of the volume fraction of the dispersed solid phase after evaporation of all the volatile solvents**

The term "volume fraction" VF of the dispersed solid phase means the percentage of the total volume V of all the solid particles present in the non-volatile fraction of the composition, relative to the total volume V' of all the compounds of the non-volatile fraction of the composition: $VF = (V/V') \times 100$

The volume V (cm$^3$) of solid particles present in the non-volatile fraction of the composition is equal to the mass m (g) of the said solid particles in the dry deposit divided by the volume mass Mv (g/cm$^3$) of the particles: $V = m/Mv$

The total volume V' (cm$^3$) of the non-volatile fraction of the composition is calculated by adding the volumes of each liquid or solid non-volatile constituent of the composition.

## Measurement of the volume masses

The volume masses of the solid particles are measured using a packing volumenometer.

A 250 ml glass measuring cylinder is filled with the powder to be measured (measuring cylinder of the type NF B 35302). The measuring cylinder was weighed beforehand on a balance with an accuracy of 0.1 g (mass M0).

The volume of powder introduced without packing, V0, should be between 240 and 250 ml.

The filled cylinder is weighed (mass M1).

The cylinder is blocked on the packing volumenometer, of the Erichsen 293 or Engelsmann JELS T2 type, and the packing machine is set for series of 2500 impacts. The series of 2500 impacts are started and the volume Vn of powder in the cylinder is noted at the end of each series.

The packing is stopped when $Vn - V(n+1) < 2 \times Vn/100$

Vn is then taken as the packing volume.

The volume mass after packing is given by the relationship $VMt = (M1-M0)/Vn$

The volume mass of the particles constituting the powder is given by the relationship $VMp = VMt/0.8$. It is this last volume mass that is used to determine the volume fraction of the solids.

The volume mass of the liquids is measured using a Gay-Lussac pycnometer.

A precision balance (accuracy of 1 mg) is used and the measurements are taken in a chamber thermostatically maintained at 25°C ($\pm$ 0.5°C). Two reference liquids having a volume mass Mv are also used, namely demineralized water ($MV = 1000$ kg/m$^3$) and heptane ($MV = 683.7$ kg/m$^3$). The volume mass of the solid particles is measured with each reference liquid.

The pycnometer and the products used to perform the measurement are placed at a temperature of 25°C. The masses cited below are expressed in kilograms.

The mass M0 of the pycnometer is measured, and the pycnometer is then filled completely with the reference liquid used, while avoiding the introduction of air bubbles.

The mass M1 of the filled pycnometer is measured.

A mixture of mass M2 of the material whose volume mass Mv2 it is desired to measure with a mass M3 of reference liquid is then prepared. The mixture is shaken and then, at the end of shaking, the pycnometer is filled with this mixture and the mass M4 of the filled pycnometer is measured. The mass M4 - M0 of the mixture present in the pycnometer is thus determined.

Since the pycnometer has a constant filling volume, the following relationship may thus be established: $(M1-M0)/Mv = (M2/Mv2 + M3/Mv) \times (M4-M0)/(M2+M3)$

This relationship makes it possible to calculate the value of the volume mass Mv2 of the solid particles, expressed in kg/m$^3$. A value of the volume mass of the solid particles is thus determined for each of the reference liquids. According to the invention, the higher value (between the volume mass measured with distilled water and the volume mass measured with heptane) is taken as the value of the volume mass for the determination of the volume fraction of the solid particles.

## Evaporation of the dispersed phase faster than evaporation of the continuous phase

According to one particular embodiment, the dispersed phase of the composition according to the invention evaporates faster than the continuous phase.

According to one preferred embodiment, the liquid fatty phase has an evaporation rate such that the weight ratio R of:

1) the ratio of the mass, at a time t = 10 minutes (corresponding to 10 minutes of drying as described above), of the liquid fraction of the dispersed aqueous phase <u>to</u> the mass, at a time t = 10 minutes, of the liquid fraction of the continuous fatty phase <u>divided by</u>

2) the ratio of the initial mass of the liquid fraction of the dispersed aqueous phase to the initial mass of the liquid fraction of the continuous fatty phase, is less than or equal to 1, in particular ranges from 0.3 to 1, preferably is less than or equal to 0.99 and in particular ranges from 0.5 to 0.99.

The ratio R is calculated using the following formula:

$$R = \frac{\dfrac{m_{disp}^{10}}{m_{cont}^{10}}}{\dfrac{m_{disp}^{0}}{m_{cont}^{0}}}$$

in which $m_{disp}^{0}$ is the initial mass of the liquid fraction of the dispersed aqueous phase of the composition $m_{disp}^{10}$ is the mass, at a time t = 10 minutes, of the liquid fraction of the dispersed aqueous phase $m_{cont}^{0}$ is the initial mass of the liquid fraction of the continuous fatty phase of the composition $m_{cont}^{10}$ is the mass, at a time t = 10 minutes, of the liquid fraction of the continuous fatty phase.

The dispersed phase of the composition evaporates faster than the continuous phase when the ratio R<1.

For the purposes of the present patent application, the term "liquid fraction of the dispersed aqueous phase or of the continuous fatty phase" means all the compounds of the dispersed phase or of the continuous phase other than the compounds of the dispersed solid phase described above.

In a non-limiting manner, the liquid fraction of the dispersed aqueous phase may comprise water, water-miscible organic solvents, certain thickeners and/or preserving agents.

In a non-limiting manner, the liquid fraction of the continuous fatty phase may comprise slow volatile oils, fast volatile oils, non-volatile oils or dry oils.

For the purposes of the present patent application, the surfactants are not included in the calculation of the liquid fraction of the dispersed aqueous phase or of the liquid fraction of the continuous fatty phase. In particular, they are not taken into account in the calculation of the ratio R.

**Additives**

The composition according to the invention may comprise at least one other common cosmetic ingredient, which may be chosen especially from hydrophilic or lipophilic gelling agents and/or thickeners, surfactants, antioxidants, fragrances, preserving agents, neutralizers, sunscreens, vitamins, moisturizers, self-tanning compounds, anti-wrinkle active agents, emollients, hydrophilic or lipophilic active agents, anti-pollution agents or free-radical scavengers, sequestrants, film-forming agents, dermorelaxing active agents, calmatives, agents for stimulating the synthesis of dermal or epidermal macromolecules and/or for preventing their degradation, anti-glycation agents, anti-irritants, desquamating agents, depigmenting agents, anti-pigmenting or pro-pigmenting agents, water-soluble or liposoluble dyes, NO-synthase inhibitors, agents for stimulating fibroblast or keratinocyte proliferation and/or keratinocyte differentiation, agents acting on the capillary circulation, agents acting on the energy metabolism of cells, and cicatrizing agents, and mixtures thereof.

According to one particular embodiment, the compositions according to the invention are liquid compositions.

For the purposes of the present patent application, the term "liquid composition" means a composition that flows under its own weight at room temperature.

The invention is illustrated in greater detail in the examples that follow.

Example 1: Water-in-oil emulsion

A foundation in the form of a water-in-oil emulsion having the composition below was prepared:

| Composition | Weight % |
|---|---|
| C8-C22 dimethicone copolyol (Abil EM 90 from the company Goldschmidt) | 3 |
| Mixture of glyceryl tristearate and of acetylated ethylene glycol (Unitwix from the company- United Guardian) | 0.5 |
| Mixture of polydiphenyldimethylsiloxane and of dimethylpolysiloxane (Mirasil C-DPDM from the company Rhodia) | 4 |
| Mixture of oxyethylenated and oxypropylenated polydimethylsiloxane, cyclopentadimethylsiloxane and water (10/88/2) (DC5225C from the company Dow Corning) | 2.6 |

(continued)

| Composition | Weight % |
|---|---|
| Cyclopentasiloxane | 7 |
| Isononyl isononanoate | 5 |
| Cyclohexasiloxane | 11 |
| Nylon powder | 3 |
| Coated titanium oxide | 12 |
| Coated yellow iron oxide | 2.2 |
| Coated brown iron oxide | 0.5 |
| Coated ultramarine blue | 0.4 |
| Magnesium sulfate | 0.7 |
| Hollow polymer microspheres such as those of polyvinylidene chloride/acrylonitrile (Expancel 551 DE from the company Expancel) | 0.5 |
| Polymethylsilsesquioxane resin microbeads (Tospearl 145 A from the company GE Toshiba Silicones) | 2 |
| UV-screening agent | 1 |
| Preserving agents | qs |
| Water | qs 100 |

This foundation was applied to the skin: it spreads easily, without any sensation of discomfort, and leads to a uniform makeup that does not transfer.

Example 2: Water-in-oil emulsion

A foundation is prepared in the form of a water-in-oil emulsion having the following composition:

| Composition | % |
|---|---|
| C8-C22 dimethicone copolyol (Abil EM 90 from the company Goldschmidt) | 3 |
| Mixture of glyceryl tristearate and of acetylated ethylene glycol (Unitwix from the company United Guardian) | 1 |
| Glycerol | 4 |
| Propylene glycol | 4 |
| Isohexadecane | 11 |
| Isododecane | 4 |
| Cyclopentasiloxane | 12.7 |
| Isononyl isononanoate | 5 |
| Nylon powder | 6 |
| Coated titanium oxide | 12 |
| Coated yellow iron oxide | 3 |
| Coated brown iron oxide | 1 |
| Water | qs 100 |

Example 3: Water-in-oil emulsion

A foundation is prepared in the form of a water-in-oil emulsion having the following composition:

| Composition | % |
|---|---|
| C8-C22 dimethicone copolyol (Abil EM 90 from the company Goldschmidt) | 3 |
| Mixture of glyceryl tristearate and of acetylated ethylene glycol (Unitwix from the company United Guardian) | 1 |
| Glycerol | 4 |
| Propylene glycol | 4 |
| Isohexadecane | 11 |
| Isododecane | 4 |
| Cyclopentasiloxane | 12.7 |
| Dicaprylyl carbonate | 5 |
| Coated titanium oxide | 12 |
| Coated yellow iron oxide | 3 |
| Coated brown iron oxide | 1 |
| Polymethylsilsesquioxane resin microbeads (Tospearl 145 A from the company GE Toshiba Silicones) | 6 |
| Water | qs 100 |

Example 4: Water-in-oil emulsion

A foundation is prepared in the form of: a water-in-oil emulsion having the following composition:

| Composition | % |
|---|---|
| C8-C22 dimethicone copolyol (Abil EM 90 from the company Goldschmidt) | 3 |
| Mixture of glyceryl tristearate and of acetylated ethylene glycol (Unitwix from the company United Guardian) | 1 |
| Glycerol | 4 |
| Propylene glycol | 4 |
| Isohexadecane | 11 |
| Isododecane | 4 |
| Cyclopentasiloxane | 12.7 |
| Dicaprylyl ether | 5 |
| Nylon powder | 2 |
| Coated titanium oxide | 12 |
| Coated yellow iron oxide | 3 |
| Coated brown iron oxide | 1 |
| Hollow polymer microspheres such as those of polyvinylidene chloride/acrylonitrile (Expancel 551 DE from the company Expancel) | 0.5 |
| Polymethylsilsesquioxane resin microbeads (Tospearl 145 A from the company GE Toshiba Silicones) | 2 |
| Water | qs 100 |

**Claims**

1.  Cosmetic composition for making up or caring for the skin, in the form of a water-in-oil emulsion comprising an aqueous phase, at least 5% by weight, relative to the total weight of the composition, of a dispersed solid phase and a liquid fatty phase having at least one non-volatile fraction, the liquid fatty phase comprising:

- at least two oils chosen from slow volatile oils with an evaporation rate of between 0.002 and 0.05 mg/cm$^2$/minute according to the protocol defined in the description and dry oils chosen from oils with a viscosity of less than or equal to 10 Cps, a surface tension of between 21 and 31 mN/m and an evaporation rate of less than 0.002 mg/cm$^2$/minute according to the protocol defined in the description,

the liquid fatty phase having an evaporation rate such that the weight ratio of the total mass of the liquid fatty phase after 10 minutes of drying according to the protocol defined in the description, ie $M = \sum_i m_i(t)$

at t=10 minutes, to the initial liquid fatty phase ie $M = \sum_i m_i(t)$ at t= 0 minutes, ranges from 0.75 to 1, wherein

i represents each solvent

$m_i(t)$ represents the remaining mass of each solvent i per unit area at time t according to the following equations

$$m_i(t) = m_i(0) - v_i.t. \quad if \quad t < \frac{m_i(0)}{v_i}$$

$$m_i(t) = 0 \quad if \quad t \geq \frac{m_i(0)}{v_i}$$

wherein $v_i$ represents the evaporation rate measured according to the protocol defined in the description and expressed in mg of oil evaporated per unit area

(cm$^2$) and per unit time (minutes).

and

the aqueous phase and the liquid fatty phase being such that the weight ratio R of:

    1) the ratio of the mass, at a time t = 10 minutes, of the liquid fraction of the dispersed aqueous phase to the mass, at a time t = 10 minutes, of the liquid fraction of the continuous fatty phase measured according to the protocol defined in the description <u>divided by</u>

    2) the ratio of the initial mass of the liquid fraction of the dispersed aqueous phase to the initial mass of the liquid fraction of the continuous fatty phase measured according to the protocol defined in the description, is less than or equal to 1,

    **characterized in that** the dry oil is present in a content ranging from 50% to 100% by weight, preferably ranging from 70% to 99% by weight, more preferably ranging from 60% to 98% by weight and more preferentially ranging from 90% to 97% by weight relative to the total weight of the non-volatile fraction of the liquid fatty phase, and is chosen from:

- isopropyl myristate, isopropyl palmitate, 2-ethylhexyl benzoate, isodecyl neopentanoate, 2-ethylhexyl 2-ethylhexanoate, isononyl isononanoate,
- dicaprylyl, ether, dicaprylyl carbonate,
- 2-diethylhexyl carbonate,
and mixtures thereof.

2. Composition according to the preceding claim, **characterized in that** the slow volatile oil with an evaporation rate of between 0.002 and 0.05 mg/cm$^2$/minute is chosen from isohexadecane, cyclohexasiloxane, diethyldodecane and hexyl trimethicone, and mixtures thereof.

3. Composition according to either of the preceding claims, **characterized in that** the slow volatile oil with an evaporation rate of between 0.002 and 0.05 mg/cm$^2$/minute is present in a content ranging from 1% to 95% by weight, preferably ranging from 2% to 50% by weight and more preferentially from 3% to 20% by weight relative to the total weight of the composition.

4. Composition according to any one of the preceding claims, **characterized in that** the calculated weight ratio of the liquid fatty phase remaining in the composition after 10 minutes of drying, to the initial liquid fatty phase, ranges from 0.75 to 0.9 and preferably from 0.8 to 0.9.

5. Composition according to any one of the preceding claims, **characterized in that** the weight ratio R ranges from 0.3 to 1, preferably is less than or equal to 0.99, in particular ranges from 0.5 to 0.99,

6. Composition according to any one of the preceding claims, **characterized in that** the non-volatile fraction of the liquid fatty phase comprises, in addition to the dry oil(s), at least one additional non-volatile oil.

7. Composition according to the preceding claim, **characterized in that** the additional non-volatile oil is present in a content of less than or equal to 7%, in particular ranging from 0.5% to 7% by weight, preferably from 1% to 5% by weight and more preferentially from 1% to 2% by weight relative to the total weight of the composition.

8. Composition according to any one of the preceding claims, **characterized in that** the liquid fatty phase comprises, in addition to the slow volatile oil with an evaporation rate of between 0.002 and 0.05 mg/cm$^2$/minute, at least one fast volatile oil chosen from volatile oils with an evaporation rate of greater than or equal to 0.05 mg/cm$^2$/minute, in particular greater than or equal to 0.054 mg/cm$^2$/minute.

9. Composition according to either of Claims 7 and 8, **characterized in that** the fast volatile oils are present in a content of less than or equal to 64% by weight, in particular ranging from 1% to 64% by weight, preferably of less than or equal to 50% by weight, in particular ranging from 2% to 50% by weight, more preferably less than or equal to 30% by weight, in particular ranging from 3% to 30% by weight, more preferentially less than or equal to 20% by weight and in particular ranging from 4% to 20% by weight relative to the total weight of the composition.

10. Composition according to any one of Claims 7 to 9, **characterized in that** the volatile fraction of the liquid fatty phase is present in a content ranging from 1% to 50% by weight, preferably from 3% to 30% by weight and more preferentially from 5% to 20% by weight relative t.o the total weight of the composition.

11. Composition according to any one of the preceding claims, **characterized in that** the said aqueous phase is present in a content ranging from 0.5% to 50% by weight, preferably from 1% to 40% by weight and more preferentially from 5% to 30% by weight relative to the total weight of the composition.

12. Composition according to any one of the preceding claims, **characterized in that** the dispersed solid phase comprises particles that are solid at 25°C and at atmospheric pressure.

13. Composition according to any one of the preceding claims, **characterized in that** the dispersed solid phase comprises pigments, nacres and/or fillers.

14. Composition according to any one of the preceding claims, **characterized in that** the dispersed solid phase is present in a content ranging from 5% to 50% by weight, better still from 10% to 45% by weight and even better still from 15% to 40% by weight relative to the total weight of the composition.

15. Composition according to any one of the preceding claims, **characterized in that** the volume fraction of the dispersed solid phase is greater than or equal to 55% by volume, relative to the total volume of the non-volatile fraction of the composition, in particular ranging from 55% to 98% by volume, preferably greater than or equal to 60% by volume, in particular ranging from 60% to 90%, more preferentially greater than or equal to 65% by volume and in particular ranging from 65% to 85% by volume.

16. Process for making up or caring for the skin, comprising the application to the skin of a composition according to any one of Claims 1 to 15.
evaporation rate of less than 0.002 mg/cm$^2$/minute, the liquid fatty phase having an evaporation rate such that the weight ratio of the liquid fatty phase after 10 minutes of drying, to the initial liquid fatty phase, ranges from 0.75 to 1, and the aqueous phase and the liquid fatty phase being such that the weight ratio R of:

1) the ratio of the mass, at a time t = 10 minutes, of the liquid fraction of the dispersed aqueous phase <u>to</u> the mass, at a time t - 10 minutes, of the liquid fraction of the continuous fatty phase
<u>divided by</u>
2) the ratio of the initial mass of the liquid fraction of the dispersed aqueous phase to the initial mass of the liquid fraction of the continuous fatty phase, is less than or equal Lo 1.

The invention also relates to the use of the said composition for obtaining a uniform makeup and/or a makeup that does not transfer.

**Patentansprüche**

**1.** Kosmetische Zusammensetzungen zum Makeup oder zur Pflege der Haut in der Form einer Wasser-in-Öl-Emulsion, umfassend eine wässrige Phase, wenigstens 5 Gew.-%, bezogen auf das Gesamtgewicht der Zusammensetzung, an einer dispergierten festen Phase und eine flüssige Fettphase mit wenigstens einer nichtflüchtigen Fraktion, wobei die flüssige Fettphase umfasst:

- wenigstens zwei Öle, ausgewählt aus langsam flüchtigen Ölen mit einer Verdunstungsrate zwischen 0,002 und 0,05 mg/cm2/Minute gemäß dem in der Beschreibung definierten Protokoll, und trockenen Ölen, ausgewählt aus Ölen mit einer Viskosität von kleiner als oder gleich 10 cps, einer Oberflächenspannung zwischen 21 und 31 mN/m und einer Verdunstungsrate kleiner als 0,002 mg/cm2/Minute gemäß dem in der Beschreibung definierten Protokoll,
wobei die flüssige Fettphase eine derartige Verdunstungsrate aufweist, dass das Gewichtsverhältnis der Gesamtmasse der flüssigen Fettphase nach 10 Minuten Trocknen gemäß dem in der Beschreibung definierten

Protokoll, d. h. $M = \sum_i m_i(t)$ bei t = 10 Minuten, zu der flüssigen Fettphase zu Anfang, d. h.

$M = \sum_i m_i(t)$ bei t = 0 Minuten, im Bereich von 0,75 bis 1 liegt,
wobei
i jedes Lösungsmittel darstellt,
mi(t) die verbleibende Masse jedes Lösungsmittels i pro Flächeneinheit zu der Zeit t gemäß folgenden Gleichungen darstellt,

$$m_i(t) = m_i(0) - v_i \cdot t, \text{ wenn } t < \frac{m_i(0)}{v_i}$$

$$m_i(t) = 0 \text{ wenn } t \geq \frac{m_i(0)}{v_i}$$

wobei vi die Verdunstungsrate darstellt, gemessen gemäß dem in der Beschreibung definierten Protokoll, und ausgedrückt in mg an pro Flächeneinheit (cm2) und Zeiteinheit (Minuten) verdunstetem Öl,
und
die wässrige Phase und die flüssige Fettphase derartig sind, dass das Gewichtsverhältnis R von

1) dem Verhältnis der Masse bei der Zeit t = 10 Minuten der flüssigen Fraktion der dispergierten wässrigen Phase zu der Masse bei der Zeit t = 10 Minuten der flüssigen Fraktion der kontinuierlichen Fettphase, gemessen gemäß dem in der Beschreibung definierten Protokoll,
geteilt durch
2) das Verhältnis der Anfangsmasse der flüssigen Fraktion der dispergierten wässrigen Phase zu der Anfangsmasse der flüssigen Fraktion der kontinuierlichen Fettphase, gemessen gemäß dem in der Beschreibung definierten Protokoll, kleiner als oder gleich 1 ist,
**dadurch gekennzeichnet, dass** das trockene Öl in einem Gehalt im Bereich von 50 Gew.-% bis 100 Gew.-%, vorzugsweise im Bereich von 70 Gew.-% bis 99 Gew.-%, bevorzugter im Bereich von 80 Gew.-% bis 98 Gew.-%, noch bevorzugter im Bereich von 90 Gew.-% bis 97 Gew.-%, bezogen auf das Gesamtgewicht der nichtflüchtigen Fraktion der flüssigen Fettphase, vorhanden ist und ausgewählt ist aus:

- Isopropylmyristat, Isopropylpalmitat, 2-Ethylhexylbenzoat, Isodecylneopentanoat, 2-Ethylhexyl-2-ethylhexanoat, Isononylisononanoat,
- Dicaprylylether, Dicaprylylcarbonat,
- 2-Diethylhexylcarbonat
und Gemischen davon.

**2.** Zusammensetzung gemäß dem vorstehenden Anspruch, **dadurch gekennzeichnet, dass** das langsam flüchtige Öl mit einer Verdunstungsrate zwischen 0,002 und 0,05 mg/cm2/Minute ausgewählt ist aus Isohexadecan, Cyclohexasiloxan, Diethyldodecan und Hexyltrimethicon und Gemischen davon.

**3.** Zusammensetzung gemäß einem der vorstehenden Ansprüche, **dadurch gekennzeichnet, dass** das langsam flüchtige Öl mit einer Verdunstungsrate zwischen 0,002 und 0,05 mg/cm2/Minute in einem Gehalt im Bereich von 1 Gew.-% bis 95 Gew.-%, vorzugsweise im Bereich von 2 Gew.-% bis 50 Gew.-%, bevorzugter von 3 Gew.-% bis 20 Gew.-%, bezogen auf das Gesamtgewicht der Zusammensetzung, vorhanden ist.

**4.** Zusammensetzung gemäß einem der vorstehenden Ansprüche, **dadurch gekennzeichnet, dass** das berechnete Gewichtsverhältnis der flüssigen Fettphase, die nach 10 Minuten Trocknen in der Zusammensetzung verbleibt, zu der Anfangsmenge der flüssigen Fettphase im Bereich von 0,75 bis 0,9 liegt, vorzugsweise von 0,8 bis 0,9.

**5.** Zusammensetzung gemäß einem der vorstehenden Ansprüche, **dadurch gekennzeichnet, dass** das Gewichtsverhältnis R im Bereich von 0,3 bis 1 liegt, vorzugsweise kleiner als oder gleich 0,99 ist, insbesondere im Bereich von 0,5 bis 0,99 liegt.

**6.** Zusammensetzung gemäß einem der vorstehenden Ansprüche, **dadurch gekennzeichnet, dass** die nichtflüchtige Fraktion der flüssigen Fettphase zusätzlich zu dem/den trockenen Öl(en) wenigstens ein zusätzliches nichtflüchtiges Öl umfasst.

**7.** Zusammensetzung gemäß dem vorstehenden Anspruch, dadurch gekenntzeichnet, dass das zusätzliche nichtflüchtige Öl in einem Gehalt von kleiner als oder gleich 7 %, insbesondere im Bereich von 0,5 Gew.-% bis 7 Gew.-%, vorzugsweise von 1 Gew.-% bis 5 Gew.-%, bevorzugter von 1 Gew.-% bis 2 Gew.-%, bezogen auf das Gesamtgewicht der Zusammensetzung, vorhanden ist.

**8.** Zusammensetzung gemäß einem der vorstehenden Ansprüche, **dadurch gekennzeichnet, dass** die flüssige Fettphase zusätzlich zu dem langsam flüchtigen Öl mit einer Verdunstungsrate zwischen 0,002 und 0,05 mg/cm2/Minute wenigstens ein schnell flüchtiges Öl umfasst, ausgewählt aus flüchtigen Ölen mit einer Verdunstungsrate von größer als oder gleich 0,05 mg/cm2/Minute, insbesondere größer als oder gleich 0,054 mg/cm2/Minute.

**9.** Zusammensetzung gemäß einem der Ansprüche 7 und 8, **dadurch gekennzeichnet, dass** die schnell flüchtigen Öle in einem Gehalt von kleiner als oder gleich 64 Gew.-%, insbesondere im Bereich von 1 Gew.-% bis 64 Gew.-%, vorzugsweise kleiner als oder gleich 50 Gew.-%, insbesondere im Bereich von 2 Gew.-% bis 50 Gew.-%, bevorzugter kleiner als oder gleich 30 Gew.-%, insbesondere im Bereich von 3 Gew.-% bis 30 Gew.-%, bevorzugter kleiner als oder gleich 20 Gew.-% und insbesondere im Bereich von 4 Gew.-% bis 20 Gew.-%, bezogen auf das Gesamtgewicht der Zusammensetzung, vorhanden sind.

**10.** Zusammensetzung gemäß einem der Ansprüche 7 bis 9, **dadurch gekennzeichnet, dass** die flüchtige Fraktion der flüchtigen Fettphase in einem Gehalt im Bereich von 1 Gew.-% bis 50 Gew.-%, vorzugsweise von 3 Gew.-% bis 30 Gew.-% und bevorzugter von 5 Gew.-% bis 20 Gew.-%, bezogen auf das Gesamtgewicht der Zusammensetzung, vorhanden ist.

**11.** Zusammensetzung gemäß einem der vorstehenden Ansprüche, **dadurch gekennzeichnet, dass** die wässrige Phase in einem Gehalt im Bereich von 0,5 Gew.-% bis 50 Gew.-%, vorzugsweise von 1 Gew.-% bis 40 Gew.-% und bevorzugter von 5 Gew.-% bis 30 Gew.-%, bezogen auf das Gesamtgewicht der Zusammensetzung, vorhanden ist.

**12.** Zusammensetzung gemäß einem der vorstehenden Ansprüche, **dadurch gekennzeichnet, dass** die dispergierte feste Phase Partikel umfasst, die bei 25 °C und atmosphärischem Druck fest sind.

**13.** Zusammensetzung gemäß einem der vorstehenden Ansprüche, **dadurch gekennzeichnet, dass** die dispergierte feste Phase Pigmente, Perlmutt und/oder Füllstoffe umfasst.

**14.** Zusammensetzung gemäß einem der vorstehenden Ansprüche, **dadurch gekennzeichnet, dass** die dispergierte feste Phase in einem Gehalt im Bereich von 5 Gew.-% bis 50 Gew.-%, besser von 10 Gew.-% bis 45 Gew.-% und noch besser von 15 Gew.-% bis 40 Gew.-%, bezogen auf das Gesamtgewicht der Zusammensetzung, vorhanden ist.

**15.** Zusammensetzung gemäß einem der vorstehenden Ansprüche, **dadurch gekennzeichnet, dass** der Volumenanteil der dispergierten festen Phase größer als oder gleich 55 Vol.-% ist, bezogen auf das Gesamtvolumen der nichtflüchtigen Fraktion der Zusammensetzung, insbesondere im Bereich von 55 Vol.-% bis 98 Vol.-% liegt, vorzugsweise größer als oder gleich 60 Vol.-% ist, insbesondere im Bereich von 60 Vol.-% bis 90 Vol.-% liegt, bevorzugter größer als oder gleich 65 Vol.-% ist, insbesondere im Bereich von 65 Vol.-% bis 85 Vol.-% liegt.

**16.** Verfahren zum Makeup oder zur Pflege der Haut, umfassend das Aufbringen einer Zusammensetzung gemäß einem der Ansprüche 1 bis 15 auf die Haut.

**Revendications**

**1.** Composition cosmétique de maquillage ou de soin de la peau sous forme d'émulsion eau-dans-huile comprenant une phase aqueuse, au moins 5 % en poids, par rapport au poids total de la composition, d'une phase solide dispersée et une phase grasse liquide ayant au moins une fraction non volatile, la phase grasse liquide comprenant :

- au moins deux huiles choisies parmi les huiles volatiles lentes ayant une vitesse d'évaporation comprise entre 0,002 et 0,05 mg/cm2/min selon le protocole défini dans la description et des huiles sèches choisies parmi les huiles ayant une viscosité inferieure ou égale à 10 Cps, une tension de surface comprise entre 21 et 31 mN/m et une vitesse d'évaporation inférieure à 0,002 mg/cm2/min selon le protocole défini dans la description, la phase grasse liquide ayant une vitesse d'évaporation telle que le rapport pondéral de la masse totale de la phase grasse liquide après 10 minutes de séchage selon le protocole défini dans la description, soit

$$M = \sum_i m_i(t)$$ à t = 10 min, sur la phase grasse liquide initiale, soit

$$M = \sum_i m_i(t)$$ à t = 0 min, va de 0,75 à 1,

où
i représente chaque solvant
mi(t) représente la masse restante de chaque solvant i par unité de surface au temps t selon les équations suivantes

$$mi(t) = mi(0) - vi.t. \text{ si } \quad t < \frac{m_i(0)}{v_i}$$

$$mi(t) = 0 \quad \text{si} \quad t \geq \frac{m_i(0)}{v_i}$$

où vi représente la vitesse d'évaporation mesurée selon le protocole défini dans la description et exprimée en mg d'huile évaporée par unité de surface (cm2) et par unité de temps (minutes),
et
la phase aqueuse et la phase grasse liquide étant telles que le rapport pondéral R de
1) le rapport de la masse, à un temps t = 10 min, de la fraction liquide de la phase aqueuse dispersée sur la masse, à un temps t = 10 min, de la fraction liquide de la phase grasse continue, mesuré selon le protocole défini dans la description,
divisé par
2) le rapport de la masse initiale de la fraction liquide de la phase aqueuse dispersée sur la masse initiale de la fraction liquide de la phase grasse continue, mesuré selon le protocole défini dans la description, est inférieur ou égal à 1,
**caractérisée en ce que** l'huile sèche est présente en une teneur allant de 50 à 100 % en poids, par rapport au poids total de la fraction non volatile de la phase grasse liquide, de préférence allant de 70 à 99 % en poids, de préférence encore allant de 80 à 98 % en poids, et plus préférentiellement allant de 90 à 97 % en poids, et est choisie parmi :

- le myristate d'isopropyle, le palmitate d'isopropyle, le benzoate d'éthyl-2 hexyle, le néopentanoate d'isodécyle, l'éthyl-2 hexanoate d'éthyl-2 hexyl,

l'isononanoate d'isononyle,

- le dicaprylyl éther, le dicaprylyl carbonate,
- le diéthyl-2 hexyl carbonate,

et leurs mélanges.

2. Composition selon la revendication précédente, **caractérisée en ce que** l'huile volatile lente ayant une vitesse d'évaporation comprise entre 0,002 et 0,05 mg/cm2/min est choisie parmi l'isohexadécane, le cyclohexasiloxane, le diéthyldodécane, l'hexyltriméthicone, et leurs mélanges.

3. Composition selon l'une quelconque des revendications précédentes, **caractérisée en ce que** l'huile volatile lente ayant une vitesse d'évaporation comprise entre 0,002 et 0,05 mg/cm2/min est présente en une teneur allant de 1 à 95 % en poids, par rapport au poids total de la composition, de préférence allant de 2 à 50 % en poids, et plus préférentiellement de 3 à 20 % en poids.

4. Composition selon l'une quelconque des revendications précédentes, **caractérisée en ce que** le rapport pondéral calculé de la phase grasse liquide restant dans la composition après 10 minutes de séchage sur la phase grasse liquide initiale va de 0,75 à 0,9, de préférence de 0,8 à 0,9.

5. Composition selon l'une quelconque des revendications précédentes, **caractérisée en ce que** le rapport pondéral R va de 0,3 à 1, de préférence est inférieur ou égal à 0,99, en particulier va de 0,5 à 0,99.

6. Composition selon l'une quelconque des revendications précédentes, **caractérisée en ce que** la fraction non volatile de la phase grasse liquide comprend, outre la/les huiles sèches, au moins une huile non volatile additionnelle.

7. Composition selon la revendication précédente, **caractérisée en ce que** l'huile non volatile additionnelle est présente en une teneur inférieure ou égale à 7 %, en particulier allant de 0,5 à 7 % en poids, par rapport au poids total de la composition, de préférence de 1 à 5 % en poids, et plus préférentiellement de 1 à 2 % en poids.

8. Composition selon l'une quelconque des revendications précédentes, **caractérisée en ce que** la phase grasse liquide comprend, outre l'huile volatile lente ayant une vitesse d'évaporation comprise entre 0,002 et 0,05 mg/cm2/min, au moins une huile volatile rapide choisie parmi les huiles volatiles ayant une vitesse d'évaporation supérieure ou égale à 0,05 mg/cm2/min, en particulier supérieure ou égale à 0,054 mg/cm2/min.

9. Composition selon l'une quelconque des revendications 7 et 8, **caractérisée en ce que** les huiles volatiles rapides sont présentes en une teneur inférieure ou égale à 64 % en poids, par rapport au poids total de la composition, en particulier allant de 1 à 64 % en poids, de préférence inférieure ou égale à 50 % en poids, en particulier allant de 2 à 50 % en poids, de préférence encore inférieure ou égale à 30 % en poids, en particulier allant de 3 à 30 % en poids, et plus préférentiellement inférieure ou égale à 20 % en poids, en particulier allant de 4 à 20 % en poids.

10. Composition selon l'une quelconque des revendications 7 à 9, **caractérisée en ce que** la fraction Volatile de la phase grasse liquide est présente en une teneur allant de 1 à 50 % en poids, par rapport au poids total de la composition, de préférence de 3 à 30 % en poids, et plus préférentiellement de 5 à 20 % en poids.

11. Composition selon l'une quelconque des revendications précédentes, **caractérisée en ce que** ladite phase aqueuse est présente en une teneur allant de 0,5 à 50 % en poids, par rapport au poids total de la composition, de préférence de 1 à 40 % en poids et plus préférentiellement de 5 à 30 % en poids.

12. Composition selon l'une quelconque des revendications précédentes, **caractérisée en ce que** la phase solide dispersée comprend des particules solides à 25°C et à pression atmosphérique.

13. Composition selon l'une quelconque des revendications précédentes, **caractérisée en ce que** la phase solide dispersée comprend des pigments, des nacres et/ou des charges.

**14.** Composition selon l'une quelconque des revendications précédentes, **caractérisée en ce que** la phase solide dispersée est présente en une teneur allant de 5 à 50 % en poids, par rapport au poids total de la composition, mieux de 10 à 45 % en poids, et mieux encore de 15 à 40 % en poids.

**15.** Composition selon l'une quelconque des revendications précédentes, **caractérisée en ce que** la fraction volumique de la phase solide dispersée est supérieure ou égale à 55 % en volume, par rapport au volume total de la fraction non volatile de la composition, en particulier va de 55 à 98 % en volume, de préférence supérieure ou égale à 60 % en volume, en particulier va de 60 à 90 %, et plus préférentiellement supérieure ou égale à 65 % en volume, en particulier va de 65 à 85 % en volume.

**16.** Procédé de maquillage ou de soin de la peau comprenant l'application sur la peau d'une composition selon l'une quelconque des revendications 1 à 15.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 518773 A
- WO 9313744 A
- WO 9313745 A
- EP 293795 A

**Non-patent literature cited in the description**

- International Cosmetic Ingredient Dictionary and Handbook. The Cosmetic, Toiletry and Fragrance Association, 1997, 371-386524-528